# EUROPÄISCHE PATENTSCHRIFT

(11) **EP 2 834 831 B1**
(45) Veröffentlichungstag und Bekanntmachung des Hinweises auf die Patenterteilung: **12.04.2017**
(21) Anmeldenummer: 12733439.9
(22) Anmeldetag: 14.06.2012
(51) Int. Cl.: H01J 35/08, H01J 35/24

(54) **RÖNTGENSTRAHLUNGSQUELLE, VERFAHREN ZUM ERZEUGEN VON RÖNTGENSTRAHLUNG SOWIE VERWENDUNG EINER MONOCHROMATISCHE RÖNTGENSTRAHLUNG AUSSENDENDEN RÖNTGENSTRAHLUNGSQUELLE**
X-RAY SOURCE, METHOD FOR PRODUCING X-RAYS AND USE OF AN X-RAY SOURCE EMITTING MONOCHROMATIC X-RAYS
SOURCE DE RAYONS X, PROCÉDÉ DE PRODUCTION DE RAYONS X ET UTILISATION D'UNE SOURCE DE RAYONS X ÉMETTANT UN RAYONNEMENT X MONOCHROMATIQUE

(43) Veröffentlichungstag der Anmeldung: 11.02.2015
(73) Patentinhaber: Siemens Aktiengesellschaft, 80333 München (DE)
(72) Erfinder: HEID, Oliver, 91052 Erlangen (DE); HUGHES, Timothy, 91056 Erlangen (DE); SIRTL, Jennifer, 90763 Fürth (DE)
(86) Internationale Anmeldenummer: PCT/EP2012/061310
(87) Internationale Veröffentlichungsnummer: WO 2013/185827

(56) Entgegenhaltungen:
- DE-A1-102009 007 871
- US-A1- 2002 150 213
- US-B1- 6 385 295
- US-B1- 7 313 226
- KEVIN B. FOURNIER ET AL: "Efficient multi-keV x-ray sources from Ti-doped aerogel targets", PROCEEDINGS OF SPIE, Bd. 5196, 7. Januar 2004 (2004-01-07), Seiten 194-204, XP055053984, ISSN: 0277-786X, DOI: 10.1117/12.505872

## Beschreibung

Die Erfindung betrifft eine Röntgenstrahlungsquelle mit einem Gehäuse, in dem ein Target vorgesehen ist, welches unter Beschuss mit einem Elektronenstrahl Röntgenstrahlung aussenden kann. Außerdem betrifft die Erfindung ein Verfahren zum Erzeugen von Röntgenstrahlung, bei dem in dem Gehäuse einer Röntgenstrahlungsquelle ein Target mit einem Elektronenstrahl beschossen wird und Röntgenstrahlung aussendet. Im Übrigen betrifft die Erfindung eine Verwendung einer monochromatische Röntgenstrahlung aussendenden Röntgenstrahlungsquelle.

Eine Röntgenstrahlungsquelle, deren Verwendung und ein Verfahren zum Erzeugen von Röntgenstrahlung der eingangs angegebenen Art ist beispielsweise in der US 2008/0144774 A1 bekannt. Danach kann eine Röntgenstrahlungsquelle beispielsweise durch eine Anordnung von Elektroden in einem Gehäuse realisiert werden. Ein Elektronenstrahl wird in dem Gehäuse durch eine Elektrode erzeugt, die ein Potential von 0 V aufweist. Dieser Elektrode gegenüberliegend ist eine Anode angeordnet, die als Target für die Elektronenstrahlung zum Einsatz kommt. Diese liegt auf 100 kV. Hinter der Anode befindet sich weiterhin ein Kollektor, der auf einem Potential von 10 kV liegt. Trifft der Elektronenstrahl auf die Anode, so wird Röntgenstrahlung freigesetzt, die durch ein geeignetes Fenster (transparent für die Röntgenstrahlung) aus dem Gehäuse ausgekoppelt und einer Verwendung zugeführt werden kann.

Die Anode, die als Target dient, kann muss als dünnwandiges Gebilde ausgeführt sein, da nur in den ersten Atomschichten eine monochromatische Röntgenstrahlung erzeugt wird. Ein dickeres Target führt zu einer zunehmenden, unerwünschten Erzeugung von Bremsstrahlung. Beispielsweise kann diese eine Basisplatte aus Bor aufweisen, die eine Dicke zwischen 10 und 200 µm hat. Auf diese wird eine dünne Schicht Wolfram mit einer Schichtdicke von 100 bis 500 nm aufgebracht, die als Target verwendet wird. Allerdings wird die sehr dünne Wolframschicht einer hohen thermischen Belastung durch den Elektrodenstrahl ausgesetzt.
Aus dem Dokument US 2002/0150213 A1 ist eine Röntgenstrahlungsquelle mit einem Gehäuse bekannt, in dem ein Target vorgesehen ist, welches unter Beschuss mit einem Elektronenstrahl Röntgenstrahlung aussenden kann, wobei als Targetmaterial ein Material ähnlich dem der Anmeldung zum Einsatz kommt.
Die Aufgabe der Erfindung besteht darin, die eingangs angegebene Röntgenstrahlungsquelle derart zu verbessern, dass eine vergleichsweise lange Betriebsdauer der Röntgenstrahlungsquelle möglich ist, ohne dass das Target ausgewechselt werden muss. Außerdem ist es Aufgabe der Erfindung, ein Verfahren zum Betrieb der genannten Röntgenstrahlungsquelle anzugeben. Zuletzt ist es Aufgabe der Erfindung, eine Verwendung für eine solche Röntgenstrahlungsquelle aufzufinden.

Die Erfindung wird in der unabhängigen Ansprüchen definiert. Weitere Details der Erfindung sind in der abhängigen Ansprüchen enthalten. Der Erfindung nach kommt als Targetmaterial ein Aerogel zum Einsatz. Das Aerogel weist eine extrem geringe Dichte auf, indem Poren in einem Netzwerk des Aerogelmaterials mit sehr dünnen Wandungen gebildet sind (ähnlich einem Schaum). Der Elektronenstrahl der Röntgenstrahlungsquelle durchdringt dieses durch das Aerogel gebildete aktive Medium und regt die K-Schale der Atome des Aerogelmaterials an. Sobald diese von ihrem angeregten Zustand auf die K-Schale zurückkehren, wird die Energie in spezifischen Quanten als monochromatische Röntgenstrahlung abgegeben. Dabei entsteht wegen der sehr geringen Dichte des Aerogels keine oder nur sehr wenig Bremsstrahlung, wobei diese sich (wenn überhaupt) in Richtung des Elektronenstrahls ausbreitet und damit leicht von der monochromatischen Röntgenstrahlung zu separieren ist. Damit kann die ungestörte monochromatische Röntgenstrahlung einer gewünschten Nutzung zugeführt werden (beispielsweise zu einem medizinischen Zweck).

Gemäß der Erfindung ist das Aerogel auf einer Metallfolie aus einem Leichtmetall oder mehreren Leichtmetallen (einer Legierung), insbesondere Aluminium, fixiert. Das Aerogel ist aufgrund seiner äußerst geringen Dichte empfindlich gegenüber mechanischer Beanspruchung, so dass die Trägerfolie mit dessen Trägerstruktur zu dessen Stabilisierung beiträgt. Die Trägerfolie selbst muss genügend dünn ausgeführt sein, damit möglichst wenig Bremsstrahlung entsteht und diese ihre Richtung entsprechend der Ausrichtung des Elektronenstrahls beibehält. Bevorzugt sollte die Metallfolie eine Dicke von 0,5 µm bis 10 µm, bevorzugt 1 µm, aufweisen, falls kein gelochter Träger verwendet wird. Der Erfindung nach handelt es sich bei der Trägerfolie um eine gelochte Folie, so kann die Metallfolie auch deutlich dicker und damit mechanisch stabiler ausgeführt sein, da sie für die emmitierte Strahlung keinen Beitrag leistet. Dabei weist die Metallfolie vorteilhaft Löcher auf, die durch das Targetmaterial überbrückt werden. Auf diese Weise ist auch der Einsatz von dickeren Folien möglich, wobei diese eine gitterartige Stützstruktur bilden. Die Löcher können beispielsweise Rundlöcher sein, die in einem regelmäßigen Muster angeordnet sind. Auf diese Weise entsteht eine regelmäßige Gitterstruktur. Besonders vorteilhaft ist es, wenn die Löcher den Querschnitt eines regelmäßigen Sechsecks aufweisen und wabenförmig in der Metallfolie angeordnet sind. Hierbei entsteht eine Stützstruktur aus Stegen, die in einem Wabenmuster angeordnet sind (entsprechend der Aufsicht auf eine Bienenwabe). Hierbei ist bei vorteilhaft möglichst geringem Materialaufwand eine maximale Stützwirkung zu erzielen. Die Entstehung von Bremsstrahlung wird vorteilhaft dabei weitgehend vermieden. Bei der Erzeugung der monochromatischen Röntgenstrahlung wird eine thermische Zerstörung des Targets in Kauf genommen.

Wenn das Target folienförmig ist (verstärkt), ist es besonders vorteilhaft, wenn das Target als Band ausgeführt ist, welches von einer ersten Rolle abgewickelt und auf eine zweite Rolle aufgewickelt werden kann. Die bandförmige Ausgestaltung der Anode hat den großen Vorteil, dass dieses durch einfache Handhabungsschritte an dem Elektronenstrahl vorbeigeführt werden kann. Hierdurch lässt sich eine Relativbewegung zwischen dem Target und dem Elektronenstrahl erzeugen. Besonders vorteilhaft ist es, das Band in Form einer Rolle der Röntgenstrahlungsquelle zuzuführen und das verbrauchte Band auf eine entsprechende Rolle aufzuwickeln, so dass es einfach möglich ist, das Band während des Betriebes der Röntgenstrahlungsquelle in dem Gehäuse sicher aufzubewahren und zum Elektronenstrahl zu führen. Außerdem kann ein einfacher Wechsel des Bandes durch Entnehmen der Rollen erfolgen, wenn dieses aufgebraucht ist. Besonders vorteilhaft kann zu diesem Zwecke vorgesehen werden, dass die erste Rolle und die zweite Rolle in Vakuumschleusen des Gehäuses untergebracht sind. Unter einer Vakuumschleuse im Sinne der Anmeldung ist ein gesonderter abgeschlossener Raum im Gehäuse zu verstehen, welcher zum einen zum Gehäuseinneren einen Durchlass für das bandförmige Targetmaterial aufweisen. Außerdem gibt es verschließbare Schleusenöffnungen nach außen, durch die hindurch die verwendeten Rollen passen. Ein Rollenwechsel kann dann durch Fluten lediglich der zur Verfügung stehenden Schleusenkammern erfolgen, so dass der restliche Gehäuseraum des Gehäuses evakuiert bleibt. Hierzu ist zu bemerken, dass die Erzeugung von Röntgenstrahlung bevorzugt in einem evakuierten Gehäuse stattfindet. Zumindest die zweite Rolle sollte vorteilhaft auch mechanisch mit einem Antrieb gekoppelt sein, welcher vorzugsweise außen am Gehäuse befestigt ist. Die Befestigung außen am Gehäuse hat den Vorteil, dass sich der Antrieb einfacher warten lässt, da dieser leicht zugänglich ist und Wartungsarbeiten nicht die Flutung des Gehäuseraums erforderlich macht.

Eine andere Möglichkeit, eine Relativbewegung zwischen Elektronenstrahl und Targetmaterial zu gewährleisten, liegt darin, dass die Erzeugungseinrichtung für den Elektronenstrahl schwenkbar ausgeführt ist. Durch Schwenken der Erzeugungseinrichtung wandert auch der Elektronenstrahl auf dem Targetmaterial hin und her, wodurch eine gleichmäßige Beaufschlagung des gesamten Targetmaterials möglich ist. Selbstverständlich kann eine schwenkbare Erzeugungseinrichtung auch mit einem Rollenmechanismus kombiniert werden. Während der Rollenmechanismus eine Bewegung des Elektronenstrahls auf dem Band in Richtung der Wickelrichtung bewirken kann, kann die Erzeugungseinrichtung insbesondere senkrecht zur Bewegungsrichtung des Bandes schwenkbar sein. Dies gewährleistet, dass das Band auch in seiner vollen Breite genutzt werden kann, wodurch eine optimale Ausnutzung des Targetmaterials möglich wird.

Eine Ausgestaltung, die nicht Teil der Erfindung ist, sieht vor, dass das Aerogel die Form einer Stange hat und mit einer Führungsvorrichtung durch den Elektronenstrahl hindurchgeführt werden kann. Die Stange kann vorteilhaft einen Querschnitt aufweisen, welcher dazu geeignet ist, durch den Elektronenstrahl vollständig durchdrungen zu werden. Daher ist eine Führung des Elektronenstrahls bei dieser Variante nicht erforderlicht. Ist das Aerogel verbraucht, so kann die Stange durch die Führungsvorrichtung bewegt werden, so dass unverbrauchtes Material in den Einflussbereich des Elektronenstrahls gebracht werden kann. Die Stangenform lässt sich vorteilhaft sehr einfach herstellen. Vorteilhaft kann die Stangenform unter Beachtung ihrer Eigenelastizität auf einer Rolle gelagert werden, die sich am anderen Ende der Führungsvorrichtung befindet. Durch Abrollen der Rolle wird die Stange des Aerogels automatisch durch die Führungsvorrichtung nachgeführt, wobei die Führungsvorrichtung gleichzeitig zu einer Begradigung des Stangenmaterials beitragen kann.
Gemäß einer vorteilhaften Ausgestaltung der Erfindung ist vorgesehen, dass das Aerogel aus einem Material besteht, dessen K-Schale der Atome eine für die Applikation brauchbare Emmisionscharakteristik aufweist. Im Einzelnen trifft diese Definition auf folgende Leichtmetalle zu: alle Alkalimetalle, alle Erdalkalimetalle außer Radium, außerdem Scandium, Yttrium, Titan und Aluminium. Andere vorteilhafte Werkstoffgruppen zur Ausbildung des Aerogels sind Wolfram, Molybdän und die Gruppe der Lanthanoide. Im Einzelnen handelt es sich dabei um das Element Lanthan die 14 im Periodensystem auf das Lanthan folgenden Elemente.
Die Verwendung des Aerogels hat außerdem den Vorteil, dass sich durch Anregung des Targets mit dem Elektronenstrahl vorteilhaft eine monochromatische Röntgenstrahlung erzeugen lässt. Dabei handelt es sich um Röntgenstrahlung, mit nur einer Wellenlänge, was den Vorteil hat, dass sich beispielsweise Röntgenbilder mit monochromatischer Röntgenstrahlung schärfer abbilden lassen. Deswegen ist es auch eine alternative Lösung der Erfindung, diese monochromatische Röntgenstrahlung zum Durchleuchten eines Körpers zu verwenden, wobei dieser so beschaffen sein muss, dass bei der Wellenlänge der verwendeten monochromatischen Röntgenstrahlung auf der Abbildung Kontraste des Körpers erscheinen. Bei dem Körper kann es sich um ein technisches Gebilde (technischer oder unbelebter Körper) wie z. B. eine Bauteilverbindung handeln, die auf Lufteinflüsse hin untersucht werden muss. Eine andere Möglichkeit ist die Aufnahme von Röntgenbildern eines menschlichen oder tierischen Körpers.
Weitere Einzelheiten der Erfindung werden nachfolgend anhand der Zeichnung beschrieben. Gleiche oder sich entsprechende Zeichnungselemente sind in den einzelnen Figuren jeweils mit den gleichen Bezugszeichen versehen und werden nur insoweit mehrfach erläutert, wie sich Unterschiede zwischen den einzelnen Figuren ergeben. Es zeigen:
- Figur 1: schematisch die Erzeugung von monochromatischer Röntgenstrahlung in einer Folie aus einem Aerogel im schematischen Schnitt,
- Figur 2: ein Ausführungsbeispiel der erfindungsgemäßen Röntgenlichtquelle, in der die Folie gemäß Figur 1 zum Einsatz kommt, im schematischen Schnitt und
- Figur 3: Beispiel eines Targetmaterials in Form einer Stange für eine Röntgenstrahlungsquelle.

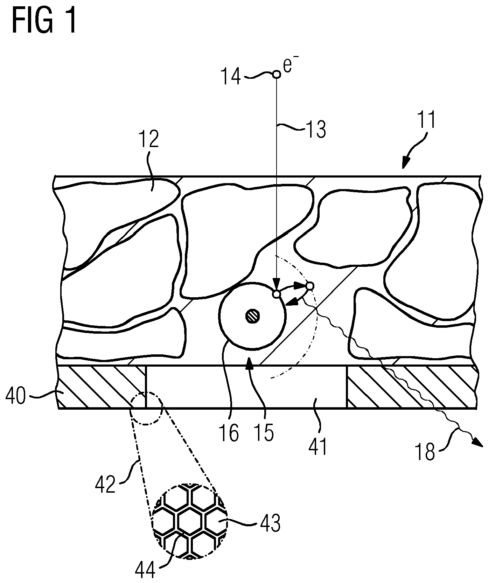

In Figur 1 ist als Target 11 ein Aerogel 12 (dargestellt als Ausschnitt) vorgesehen. Ein Elektronenstrahl 13 trifft mit Elektronen 14 auf ein Atom 15 des Targetmaterials (z. B. Lanthan). Dargestellt ist auch die K-Schale 16 des Atoms 15, wobei der Elektronenstrahl bewirkt, dass eines der Elektronen 17 der K-Schale 16 angeregt wird und auf eine andere Schale angehoben wird. Wenn diese Elektronen zurückspringen, wird hierbei monochromatische Röntgenstrahlung 18 ausgesendet.

Das Aerogel 12 ist auf einer Metallfolie 40 aufgebracht, die das Aerogel 12 stützt. Diese Metallfolie 40 weist überdies Löcher 41 auf, die in einem regelmäßigen Muster in der Metallfolie 40 angeordnet sein können, um bei möglichst großer Stützwirkung der Metallfolie 40 einen möglichst geringe Materialdichte/Atomlagenzahl zu gewährleisten. Wie einer Ausschnittsdarstellung 42 (Aufsicht auf die Metallfolie) zu entnehmen ist, können die Löcher 41 insbesondere die Form von Waben 43 aufweisen. Das Material der Metallfolie 40 ist dann in Form von Stegen 44 ausgebildet. Die Breite der Stege kann unter Berücksichtigung der erforderlichen Stützwirkung konstruktiv bestimmt werden.

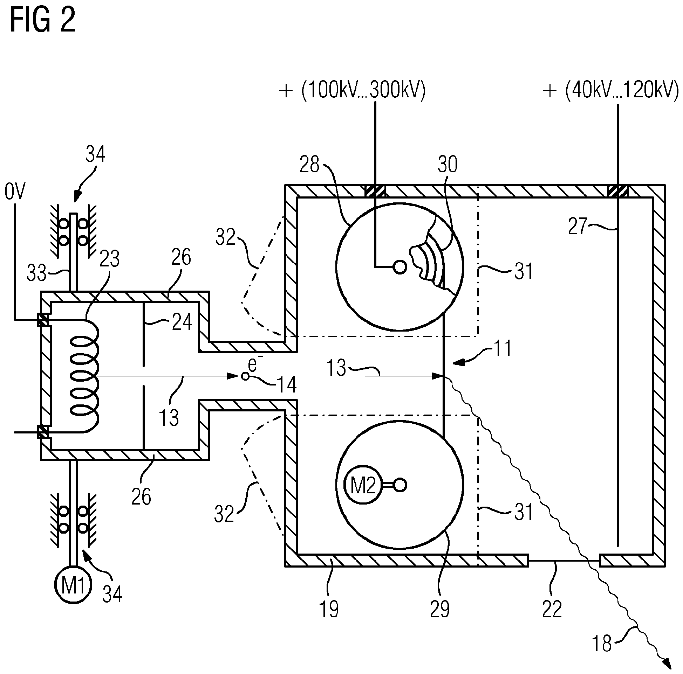

Der Figur 2 lässt sich der Aufbau der erfindungsgemäßen Röntgenstrahlungsquelle entnehmen. Die Röntgenstrahlungsquelle selbst ist in einem evakuierbaren Gehäuse 19 untergebracht, welches ein Fenster 22 aufweist. Der Elektronenstrahl trifft auf das Target 11, wobei dieses aufgrund seiner geringen Dicke kaum Energie des Elektronenstrahls absorbiert. Allerdings wird ein Teil der Energie durch eine Anregung der Atome 15 (siehe Figur 1) in der bereits beschriebenen Weise in monochromatische Röntgenstrahlung 18 umgesetzt, welche das Gehäuse durch das Fenster 22 verlassen kann.
Der das Target durchdringende Elektronenstrahl wird durch den Kollektor elektrostatisch so weit abgebremst, dass die Elektronen des Elektronenstrahls am Kollektor so niederenergetisch einschlagen, dass keine Bremsstrahlung erzeugt werden kann. Um die Elektronen 14 im Elektronenstrahl 13 genügend zu beschleunigen, kann eine sogenannte E-Gun (also eine Elektronenkanone) vorgesehen werden. Diese weist eine beheizte Kathode 23 auf, welche bei Vorliegen eines elektrischen Feldes Elektronen aussendet. Diese Elektronen werden mittels einer elektrischen Linse 24 gebündelt, damit der Elekronenstrahl eine möglichst kleine, punktförmige Strahlungsquelle auf dem Target erzeugt. Das elektrische Feld wird dadurch aufgebaut, dass das Target als Anode geschaltet ist. Diese kann mit einem Potential von 100 bis 300 kV betrieben werden, wobei zusätzlich hinter dem Target noch ein Kollektor 27 mit einem Potential von 40 bis 120 kV zum Einsatz kommt.

In dem Gehäuse sind außerdem eine erste Rolle 28 und eine zweite Rolle 29 vorgesehen. Auf der ersten Rolle 28 ist das Target gemäß Figur 1, welches in Form eines Bandes 30 vorliegt, aufgerollt und wird in nicht näher dargestellter Weise mittels eines Stellantriebs M2 (sitzt außerhalb des Gehäuses in an sich bekannter Weise auf einer Antriebswelle zur Drehung der Rolle 29) angetrieben. Dabei wird das Target 11 von der Rolle 28 abgewickelt und auf die Rolle 29 aufgewickelt. Damit ein Wechsel der Rollen 28, 29 einfach möglich ist, sind strichpunktiert angedeutete Vakuumschleusen 31 vorgesehen, so dass der restliche Raum des Gehäuses bei einem Wechsel der Rollen 28, 29 nicht geflutet werden muss. Die Rollen 28, 29 werden durch die angedeuteten Klappen 32 entnommen.

Die Elektronenkanone ist ebenfalls über eine Welle 33 schwenkbar gelagert. Ein Antrieb erfolgt über einen Motor M1. Die Welle 33 liegt parallel zur Zeichenebene in Lagern 34, so dass ein Schwenken der Elektronenkanone dazu führt, dass der Elektronenstrahl 13 über die gesamte Breite des Bandes 30 geschwenkt werden kann. Der Antrieb der Rollen 28, 29 bewirkt, dass der Elektronenstahl auch in Richtung der Längenausdehnung des Bandes 30 die Auftreffstelle auf dem Target wechseln kann.

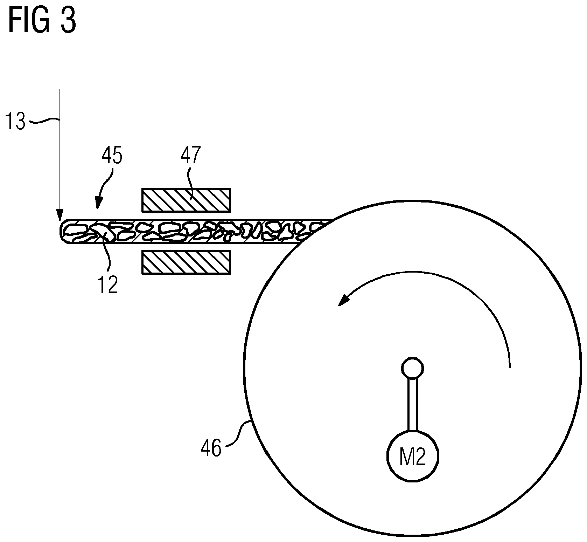

In Figur 3 ist ein Target in Form einer Stange 45 dargestellt. Dieses ist auf eine Vorratsrolle 46 mit einem genügend großen Durchmesser aufgewickelt, damit die Verformung aufgrund des Durchmessers der Rolle 46 das Aerogel 12 des Targets nicht beschädigt, insbesondere nur elastisch verformt. Durch eine Führungsvorrichtung 47 wird das Target 45 dem Elektronenstrahl 13 zugeführt. Hierbei kommt in der zu Figur 2 beschriebenen Weise der Motor M2 zum Einsatz. Eine weitere Rolle zum Aufwickeln des Targets ist im Unterschied zu Figur 2 nicht erforderlich, da dieses bei Bestrahlen mit dem Elektronenstrahl 13 verdampft. Man kann sich die Vorrichtung gemäß Figur 3 dennoch in die Röntgenstrahlungsquelle gemäß Figur 2 eingebaut vorstellen, indem diese statt der Rolle 28 verwendet wird. Die Rolle 29 wird in diesem Fall nicht verwendet.

## Patentansprüche

1. Röntgenstrahlungsquelle mit einem Gehäuse (19), in dem ein Target (11) vorgesehen ist, welches unter Beschuss mit einem Elektronenstrahl (13) Röntgenstrahlung aussenden kann,
**dadurch gekennzeichnet,**
**dass** als Targetmaterial ein Aerogel (12) zum Einsatz kommt,
**dass** das Aerogel (12) auf einer Metallfolie (40) aus einem Leichtmetall oder mehreren Leichtmetallen fixiert ist, wobei die Metallfolie (40) Löcher (41) aufweist, die durch das Targetmaterial überbrückt werden;
wobei beispielsweise das Targetmaterial aus einem Leichtmetall bestehen kann.

2. Röntgenstrahlungsquelle nach Anspruch 1,
**dadurch gekennzeichnet,**
**dass** das Aerogel (12) auf einer Metallfolie (40) aus Aluminium fixiert ist.

3. Röntgenstrahlungsquelle nach Anspruch 2,
**dadurch gekennzeichnet,**
**dass** die Metallfolie (40) eine Dicke von 0,5 µm bis 10 µm, bevorzugt 1 µm aufweist.

4. Röntgenstrahlungsquelle nach Anspruch 1,
**dadurch gekennzeichnet,**
**dass** die Löcher (41) in einem regelmäßigen Muster angeordnet sind.

5. Röntgenstrahlungsquelle nach Anspruch 4,
**dadurch gekennzeichnet,**
**dass** die Löcher (41) den Querschnitt eines regelmäßigen Sechsecks aufweisen und wabenförmig in der Metallfolie (40) angeordnet sind.

6. Röntgenstrahlungsquelle nach einem der vorangehenden Ansprüche,
**dadurch gekennzeichnet,**
**dass** das Target als Band (30) ausgeführt ist, welches von einer ersten Rolle (28) abgewickelt und auf eine zweite Rolle (29) aufgewickelt werden kann.

7. Röntgenstrahlungsquelle nach einem der vorangehenden Ansprüche,
**dadurch gekennzeichnet,**
**dass** in dem Targetmaterial ein Element aus der Reihe der Lathanoide enthalten ist.

8. Röntgenstrahlungsquelle nach einem der Ansprüche 1 bis 6,
**dadurch gekennzeichnet,**
**dass** das Targetmaterial aus einem Leichtmetall, insbesondere Aluminium besteht.

9. Verfahren zum Erzeugen von Röntgenstrahlung, bei dem in dem Gehäuse (19) einer Röntgenstrahlungsquelle ein Target (11) mit einem Elektronenstrahl (13) beschossen wird und Röntgenstrahlung aussendet,
**dadurch gekennzeichnet,**
**dass** als Target (11) ein Aerogel (12) verwendet wird, das auf einer Metallfolie (40) aus einem Leichtmetall oder mehreren Leichtmetallen fixiert ist, wobei die Metallfolie (40) Löcher (41) aufweist, die durch das Targetmaterial überbrückt werden, wobei beispielsweise das Targetmaterial aus einem Leichtmetall bestehen kann.

10. Verfahren nach Anspruch 9,
**dadurch gekennzeichnet,**
**dass** eine mit dem Target (11) erzeugte monochromatische Röntgenstrahlung einer gewünschten Nutzung zugeführt wird.

11. Verfahren nach Anspruch 9 oder 10,
**dadurch gekennzeichnet,**
**dass** neues Targetmaterial des Aerogels (12) nachgeführt wird, wenn das alte durch den Elektronenstrahl (13) verbraucht ist.

12. Verwendung einer monochromatische Röntgenstrahlung aussendenden Röntgenstrahlungslichtquelle nach einem der Ansprüche 1 bis 8 zum Durchleuchten eines Körpers, der bei der Wellenlänge der verwendeten Röntgenstrahlung differenzierbare Kontraste ausbildet.

## Claims

1. X-ray source having a housing (19) in which a target (11) is provided that can emit X-rays when being bombarded with an electron beam (13), **characterized in that** an aerogel (12) is used as the target material, **in that** the aerogel (12) is fixed on a metal foil (40) made of a light metal or a plurality of light metals, wherein the metal foil (40) has holes (41) which are bridged by the target material, wherein for example the target material can be made of a light metal.

2. X-ray source according to Claim 1, **characterized in that** the aerogel (12) is fixed on a metal foil (40) made of aluminum.

3. X-ray source according to Claim 2, **characterized in that** the metal foil (40) has a thickness of 0.5 µm to 10 µm, preferably 1 µm.

4. X-ray source according to Claim 1, **characterized in that** the holes (41) are arranged in a regular pattern.

5. X-ray source according to Claim 4, **characterized in that** the holes (41) have the cross section of a regular hexagon and are arranged in the form of honeycombs in the metal foil (40).

6. X-ray source according to one of the preceding claims, **characterized in that** the target is configured as a tape (30) which can be unwound from a first roller (28) and wound onto a second roller (29).

7. X-ray source according to one of the preceding claims, **characterized in that** an element from the series of the lanthanides is included in the target material.

8. X-ray source according to one of Claims 1 to 6, **characterized in that** the target material is made of a light metal, preferably aluminum.

9. Method for producing X-rays, in which in the housing (19) of an X-ray source, a target (11) is bombarded with an electron beam (13) and X-rays are emitted, **characterized in that** an aerogel (12) is used as the target (11), which aerogel (12) is fixed on a metal foil (40) made of a light metal or a plurality of light metals, wherein the metal foil (40) has holes (41) which are bridged by the target material, wherein for example the target material can be made of a light metal.

10. Method according to Claim 9, **characterized in that** monochromatic X-rays produced using the target (11) are supplied to a desired use.

11. Method according to Claim 9 or 10, **characterized in that** new target material of the aerogel (12) is supplied when the old material is used up by the electron beam (13).

12. Use of an X-ray light source emitting monochromatic X-rays according to one of Claims 1 to 8 for X-raying a body, which produces differentiable contrasts at the wavelength of the X-rays used.

## Revendications

1. Source de rayons X comportant un boîtier (19) dans lequel est disposée une cible (11) qui, sous bombardement avec un faisceau d'électrons (13), peut émettre des rayons X, **caractérisée en ce que** l'on utilise comme matière cible un aérogel (12), et **en ce que** l'aérogel (12) est fixé sur une feuille métallique (40) en un métal léger ou plusieurs métaux légers, dans laquelle la feuille métallique (40) comprend des trous (41) qui sont recouverts par la matière cible ; dans laquelle la matière cible peut être constituée par exemple d'un métal léger.

2. Source de rayons X selon la revendication 1, **caractérisée en ce que** l'aérogel (12) est fixé sur une feuille métallique (40) en aluminium.

3. Source de rayons X selon la revendication 2, **caractérisée en ce que** la feuille métallique (40) a une épaisseur de 0,5 µm à 10 µm, de préférence de 1 µm.

4. Source de rayons X selon la revendication 1, **caractérisée en ce que** les trous (41) sont disposés selon un motif régulier.

5. Source de rayons X selon la revendication 4, **caractérisée en ce que** les trous (41) présentent la section transversale d'un hexagone régulier et sont disposés dans la feuille métallique (40) sous forme de nids d'abeilles.

6. Source de rayons X selon l'une des revendications précédentes, **caractérisée en ce que** la cible est réalisée sous forme de bande (30) qui peut se dérouler depuis un premier rouleau (28) et être enroulée sur un second rouleau (29).

7. Source de rayons X selon l'une des revendications précédentes, **caractérisée en ce que** la matière cible contient un élément de la série des lanthanides.

8. Source de rayons X selon l'une des revendications 1 à 6, **caractérisée en ce que** la matière cible est constituée d'un métal léger, en particulier d'aluminium.

9. Procédé pour produire des rayons X, selon lequel, dans le boîtier (19) d'une source de rayons X, une cible (11) est bombardée par un faisceau d'électrons (13) et émet des rayons X, **caractérisé en ce que** l'on utilise comme cible (11) un aérogel (12) qui est fixé sur une feuille métallique (40) en un métal léger ou plusieurs métaux légers, dans lequel la feuille métallique (40) comprend des trous (41) qui sont recouverts par la matière cible, dans lequel la matière cible peut être constituée par exemple d'un métal léger.

10. Procédé selon la revendication 9, **caractérisé en ce que** des rayons X monochromatiques produits par la cible (11) sont fournis pour une utilisation souhaitée.

11. Procédé selon la revendication 9 ou 10, **caractérisé en ce que** l'on utilise une nouvelle matière cible de l'aérogel (12) quand l'ancienne a été consommée par le faisceau d'électrons (13).

12. Utilisation d'une source lumineuse de rayons X émettant des rayons X monochromatiques selon l'une des revendications 1 à 8, pour la scrutation d'un corps qui produit des contrastes pouvant être différenciés sous les longueurs d'onde des rayons X utilisés.
